# EUROPEAN PATENT SPECIFICATION

(11) **EP 3 456 208 B1**
(45) Date of publication and mention of the grant of the patent: **21.06.2023**
(21) Application number: 17795865.9
(22) Date of filing: 04.04.2017
(51) Int. Cl.: A23L 33/105, A23L 2/52, A23L 33/10, A23L 33/15, A61K 31/015, A61K 31/12, A61K 31/122, A61K 31/215, A61K 31/355, A61K 31/375, A61P 3/02, A61K 31/34, A61K 31/045

(54) **AGENT FOR IMPROVING CAROTENOID BALANCE IN BLOOD**
MITTEL ZUR VERBESSERUNG DES KAROTINOIDGLEICHGEWICHTS IM BLUT
AGENT POUR AMÉLIORER L'ÉQUILIBRE EN CAROTÉNOÏDES DANS LE SANG

(30) Priority: 13.05.2016 JP 2016096954
(43) Date of publication of application: 20.03.2019
(73) Proprietor: Glico Nutrition Co., Ltd., Osaka-shi, Osaka 555-8502 (JP)
(72) Inventor: NISHINO, Azusa, Osaka-shi Osaka 555-8502 (JP)
(74) Representative: Uexküll & Stolberg
(86) International application number: PCT/JP2017/014126
(87) International publication number: WO 2017/195504

(56) References cited:
- WO-A1-2015/046365
- WO-A1-2016/035790
- CN-A- 103 251 927
- CN-A- 103 719 301
- JP-A- 2008 532 492
- JP-A- 2009 517 043
- ANTONIO PEREZ-GALVEZ ET AL: "Incorporation of carotenoids from paprika oleoresin into human chylomicrons", BRITISH JOURNAL OF NUTRITION, vol. 89, no. 06, 1 June 2003 (2003-06-01), page 787, XP055438908, UK ISSN: 0007-1145, DOI: 10.1079/BJN2003842
- PARK, JIHYUN et al.: "Changes in carotenoids, ascorbic acids, and quality characteristics by the pickling of paprika (Capsicum annuum 1.) cultivated in Korea", J. Food Sci., vol. 76, 2011, page C1077, XP055438903,
- BALCERCZYK, ANETA et al.: "Enhanced antioxidant capacity and anti-ageing biomarkers after diet micronutrient supplementation", Molecules, vol. 19, 2014, pages 14794-14808, XP055438904,
- NISHINO, AZUSA et al.: "Accumulation of Paprika Carotenoids in Human Plasma and Erythrocytes", J. Oleo Sci., vol. 64, no. 10, 2015, pages 1135-1142, XP055438905,
- ETOH, HIDEO et al.: "Carotenoids in human blood plasma after ingesting paprika juice", Biosci. Biotechnol. Biochem., vol. 64, no. 5, May 2000 (2000-05), pages 1096-1098, XP055438907,
- HIDEKO NIHEI et al.: "Paprika Xanthophyll ga Kecchu no Kosankaryoku to Sanka Stress ni Oyobosu Eikyo", Japanese Journal of Physical Fitness and Sports Medicine, vol. 64, 2015, page 612, XP009516549, ISSN: 0039-906X
- PEREZ-GALVEZ, ANTONIO et al.: "Incorporation of carotenoids from paprika oleoresin into human chylomicrons", British Journal of Nutrition, vol. 89, no. 6, June 2003 (2003-06), pages 787-793, XP055438908,
- Anonymous: "Aka Paprika Yurai no Kenko Sozai 'PapriX' = [A healthy material derived from red paprika PapriX ", Japan Food Science, vol. 55, no. 7, 5 July 2016 (2016-07-05), pages 20-23, XP009515462, ISSN: 0368-1122

## Description

### TECHNICAL FIELD

The present invention relates to an agent for improving a carotenoid balance in blood. More specifically, the present invention relates to an agent for improving a carotenoid balance in blood which increases concentrations of xanthophylls which are normally not present in blood while inhibiting decreases in concentrations of carotenoids which are normally present in blood. The present invention also relates to a food or beverage capable of increasing concentrations of xanthophylls which are normally not present in blood while inhibiting decreases in concentrations of carotenoids which are normally present in blood.

### BACKGROUND ART

Carotenoids are natural pigment components widely distributed in plants and animals, there are great types of carotenoids, and it is said to be over 700 species. The carotenoid has antioxidant properties, and the carotenoid has a basic structure composed of a polyene moiety composed of nine conjugated double bonds and end groups attached to both ends thereof.

About 20 types of carotenoids absorbed from food are normally present in human blood, and among carotenoids which are normally present in blood (hereinafter also referred to as "normally present carotenoids"), six types, namely, β-carotene, α-carotene, β-cryptoxanthin, lutein, zeaxanthin, and lycopene are the main components, which account for 90% or more of the carotenoids in blood (see Non-Patent Document 1). These normally present carotenoids prevent lifestyle diseases, cancer, arteriosclerosis, etc. by eliminating active oxygen constantly occurring in the body, and contribute to maintenance of health.

On the other hand, among xanthophylls which are one type of carotenoids, β-cryptoxanthin, lutein, zeaxanthin, capsorubin, capsanthin, capsanthone, cucurbitaxanthin A, astaxanthin, mytiloxanthin and the like are known as components having high antioxidant properties and playing beneficial roles in maintaining health. Among these xanthophylls, β-cryptoxanthin, lutein and zeaxanthin are present in the body as normally present carotenoids, but others are normally not present in blood (hereinafter, xanthophylls which are normally not present in blood are also referred to as "normally non-present xanthophylls").

Various compositions have been conventionally proposed for efficiently ingesting normally non-present xanthophylls and maintaining health and preventing diseases. For example, Patent Document 1 discloses that a composition containing a xanthophyll such as astaxanthin is effective for improving/increasing cognitive motor function. In addition, Patent Document 2 discloses that a composition containing a xanthophyll such as astaxanthin and a peptide is effective for improving ocular regulatory dysfunction.

However, when ingesting xanthophyll alone, there is the disadvantage that the blood levels of other normally present carotenoids are lowered, the balance of blood carotenoids is impaired, and the function originally exhibited by the normally present carotenoids is attenuated. For example, Non-Patent Document 2 reports that blood levels of β-cryptoxanthin, lutein, and zeaxanthin are significantly lowered when astaxanthin alone is ingested. In addition, Non-Patent Document 3 reports that the blood levels of β-carotene, α-carotene, and β-cryptoxanthin are significantly lowered when lutein and zeaxanthin are ingested in combination. Furthermore, Non-Patent Document 4 reports that when a combination of capsanthin, capsorubin, cucurbitaxanthin A, β-cryptoxanthin, zeaxanthin, β-carotene, cryptocapsin, capsanthin epoxide and the like is ingested, blood levels of the total carotenes and lutein are significantly lowered.

Patent Document 6 discloses a combination of beta-carotene, lycopene, astaxanthine, for the treatment of the presently claimed disorders dementia, diabetes, and cataract.

Under such prior art, it has been desired to develop a composition capable of increasing the blood levels of normally non-present xanthophylls having high antioxidant properties while inhibiting decreases in the blood levels of normally present carotenoids.

### PRIOR ART DOCUMENTS

### NON-PATENT DOCUMENT

Non-Patent Document 1: Biological Functions of Carotenoids, Food and Clinical Nutrition, 2007, Vol. 2, p. 3-14, Japan Science Society of Biological Macromolecules
Non-Patent Document 2: Erythrocytes Carotenoids after Astaxanthin Supplementation in Middle-Aged and Senior Japanese Subjects. J.Oleo.Sci., 60, (10)495-499(2011)
Non-Patent Document 3: Antioxidant effect of lutein towards phospholipid hydroperoxidation in human erythrocytes. British Journal of Nutrition (2009), 2.3, 1280-1284
Non-Patent Document 4: Accumulation of paprika carotenoids in human plasma and erythrocytes, J.Oleo.Sci., 64 (10) 1135-1142 (2015)
Non-Patent Document 5: Incorporation of carotenoids from paprika oleoresin into human chylomicrons. British Journal of Nutrition (2003), vol. 89, page 787.
Non-Patent Document 6: Changes in carotenoids, ascorbic acids, and quality characteristics by the pickling of paprika (Capsicum annuum 1.) cultivated in Korea. J. Food Sci. (2011), vol. 76, page C1077.
Non-Patent Document 7: Enhanced antioxidant capacity and anti-ageing biomarkers after diet micronutrient supplementation. Molecules (2014), vol. 19, pages 14794-14808.

### PATENT DOCUMENTS

Patent Document 1: Japanese Patent Laid-open Publication No. 2009-298740
Patent Document 2: Japanese Patent Laid-open Publication No. 2008-297222
Patent Document 3: CN 103 251 927 A
Patent Document 4: WO 2015/046365 A1
Patent Document 5: Japanese Patent Laid-open Publication No. 2008-532492.
Patent Document 6: CN 103 719 301 A.

### SUMMARY OF THE INVENTION

### PROBLEMS TO BE SOLVED BY THE INVENTION

The present invention has been made in view of the above-described problems of the prior art, and it is an object of the present invention to provide an agent, a food or beverage for improving a carotenoid balance in blood which increases the blood levels of normally non-present xanthophylls while inhibiting decreases in the blood levels of normally present carotenoids.

### MEANS FOR SOLVING THE PROBLEM

The present inventor has conducted intensive studies to solve the above-mentioned problems, and has found that it is possible to increase the blood levels of normally non-present xanthophylls having high antioxidant properties while inhibiting decreases in blood levels of the normally present carotenoids and to improve the carotenoid balance in blood, by combining: (A) a specific normally non-present xanthophyll having high antioxidant properties; (B) a component corresponding to xanthophyll among normally present carotenoids; (C) a component corresponding to carotene among normally present carotenoids; and (D) vitamin E, vitamin C and/or vitamin C derivatives. More specifically, the present inventor has found that it is possible to increase the blood levels of normally non-present xanthophylls having high antioxidant properties while inhibiting decreases in the blood levels of normally present carotenoids, by combining: (A) at least one member selected from the group consisting of capsanthin, capsorubin, capsanthone, cucurbitaxanthin A, astaxanthin, mytiloxanthin, and fatty acid esters thereof; (B) at least one member selected from the group consisting of zeaxanthin, β-cryptoxanthin, lutein, and fatty acid esters thereof; (C) at least one member selected from the group consisting of β-carotene, α-carotene, and lycopene; and (D) vitamin E. The present invention has been completed by further investigation based on these findings.

The invention is defined by the claims. Any subject-matter falling outside the scope of the claims is provided for information purposes only.

Namely, the present invention provides an agent comprising:
(A) at least one member selected from the group consisting of capsanthin, capsorubin, capsanthone, cucurbitaxanthin A, astaxanthin, mytiloxanthin, and fatty acid esters thereof;
(B) at least one member selected from the group consisting of zeaxanthin, β-cryptoxanthin, lutein, and fatty acid esters thereof;
(C) at least one member selected from the group consisting of β-carotene, α-carotene, and lycopene; and
(D) vitamin E,
for use in a method of treating dementia, arteriosclerosis, hypertension, diabetes, hyperlipidemia, cataract, and cerebral stroke.

### ADVANTAGES OF THE INVENTION

The agent, food or beverage for improving the carotenoid balance in blood according to the present invention can increase the blood levels of normally non-present xanthophylls having high antioxidant properties while inhibiting decreases in the blood levels of normally present carotenoids, and therefore, it becomes possible to effectively impart antioxidant properties, provitamin A functions and the like possessed by normally non-present xanthophylls while maintaining the functions of normally present carotenoids. In addition, since the agent, food or beverage for improving the carotenoid balance in blood according to the present invention can be provided in the form of a supplement, a drink, etc., it becomes possible to maintain health by carotenoids and prevent diseases on a routine basis conveniently.

### EMBODIMENTS OF THE INVENTION

### 1. Agent for improving carotenoid balance in blood

The agent for improving the carotenoid balance in blood according to the present invention comprises: (A) at least one member selected from the group consisting of capsanthin, capsorubin, capsanthone, cucurbitaxanthin A, astaxanthin, mytiloxanthin, and fatty acid esters thereof (also referred to as the component (A)); (B) at least one member selected from the group consisting of zeaxanthin, β-cryptoxanthin, lutein, and fatty acid esters thereof (also referred to as the component (B)); (C) at least one member selected from the group consisting of β-carotene, α-carotene, and lycopene (also referred to as the component (C)); and (D) vitamin E (also referred to as the component (D)). Hereinafter, the agent for improving the carotenoid balance in blood according to the present invention will be described in detail.

### Component (A)

The component (A) used in the present invention is at least one member selected from the group consisting of capsanthin, capsorubin, capsanthone, cucurbitaxanthin A, astaxanthin, mytiloxanthin, and fatty acid esters thereof, which are normally non-present xanthophylls having high antioxidant properties (scavenging activities of singlet oxygen).

Capsanthin is a xanthophyll contained in red fruits of plants of the genus Capsicum such as red-color paprika, red bell pepper, and red pepper. The capsanthin used in the present invention may be extracted from these plants or chemically synthesized.

Capsorubin is a xanthophyll contained in red fruits of plants of the genus Capsicum such as red-color paprika, red bell pepper, and red pepper. The capsorubin used in the present invention may be extracted from these plants or chemically synthesized.

Capsanthone is an oxide of capsanthin and is a xanthophyll contained in red fruits of plants of the genus Capsicum such as red-color paprika, red bell pepper, and red pepper. The capsanthone used in the present invention may be extracted from these plants, obtained by subjecting capsanthin to oxidation treatment or chemically synthesized.

Cucurbitaxanthin A is a xanthophyll contained in red fruits of plants of the genus Capsicum such as red-color paprika, red bell pepper, and red pepper; and plants such as pumpkin. The cucurbitaxanthin A used in the present invention may be extracted from these plants or chemically synthesized.

Astaxanthin is a xanthophyll contained in Crustacea such as shrimp, and crab; fish and shellfish such as salmon, sea bream, carp, goldfish, starfish, barnacle, krill, squid, and octopus; plants such as petals of Adonis ramosa; Aves such as chicken; microorganisms such as green algae (Haematococcus pluvialis), Euglena (Euglena heliorubescens), Chlorella (Chlorela zofingiensis), red yeast (Phaffia rhodozyma), thermophilic bacteria (Meiothermus ruber) and the like. The astaxanthin used in the present invention may be extracted from these natural products, obtained by culturing astaxanthin-producing microorganisms or chemically synthesized.

Mytiloxanthin is a xanthophyll contained in xanthophylls and the like contained in shellfish such as mussel, and oyster. The mytiloxanthin used in the present invention may be extracted from these shellfish or chemically synthesized.

In addition, the xanthophyll may be an ester in which fatty acid(s) is/are ester-bonded. The ester of the xanthophyll may be a monoester in which one fatty acid is ester-bonded or a diester in which two fatty acids are ester-bonded. The fatty acid bonded in the ester of the xanthophyll may be either a saturated fatty acid or an unsaturated fatty acid, and the fatty acid may be either linear or branched. The number of carbon atoms of the fatty acid bonded in the ester of the xanthophyll is not particularly limited, but it is, for example, about 1 to 20, preferably about 2 to 18, and more preferably about 12 to 18. In the case where the ester of the xanthophyll is a diester, the two fatty acids bonded may have the same structure or different structures. Hereinafter, the concept that encompasses capsorubin and/or a fatty acid ester thereof may be referred to as "capsorubins" in some cases. Likewise, with respect to capsanthin and/or a fatty acid ester thereof, capsanthone and/or a fatty acid ester thereof, and cucurbitaxanthin A and/or a fatty acid ester thereof, similarly, they may be referred to as "capsanthins", "capsanthones", and "cucurbitaxanthin As", respectively, in some cases.

In the agent for improving the carotenoid balance in blood according to the present invention, as the component (A), one type selected from capsanthin, capsorubin, capsanthone, cucurbitaxanthin A, astaxanthin, mytiloxanthin and fatty acid esters thereof may be used or two or more types of them may be used in combination.

Among these components (A), from the viewpoint of more effectively inhibiting the decreases in the blood levels of the normally present carotenoids and increasing the blood levels of the normally non-present xanthophylls, for example, preferably, at least one type of capsanthins, capsorubins, capsanthones, and cucurbitaxanthin As is used; more preferably at least one type of capsanthins, capsorubins, and cucurbitaxanthin As is used; and particularly preferably a combination of capsanthins, capsorubins, and cucurbitaxanthin As is used.

In the case where a combination of capsanthins, capsorubins, and cucurbitaxanthin As is used as the component (A), the ratio of them is not particularly limited. Examples of the ratio include, per 100 parts by mass of capsanthins in terms of free capsanthin weight, 1 to 1000 parts by mass, preferably 2 to 100 parts by mass and more preferably 5 to 30 parts by mass of capsorubins in terms of free capsorubin weight; and 1 to 1000 parts by mass, preferably 2 to 100 parts by mass and more preferably 5 to 30 parts by mass of cucurbitaxanthin As in terms of free cucurbitaxanthin A weight. In the present specification, capsanthins in terms of free capsanthin weight refers to the value of the weight of itself in the case of capsanthin, and in the case of fatty acid ester(s) of capsanthin, it refers to the value converted to the weight of capsanthin (free form) per se, excluding the fatty acid moiety. The same applies to capsorubins in terms of capsorubin weight and cucurbitaxanthin As in terms of cucurbitaxanthin A weight.

### Component (B)

The component (B) used in the present invention is at least one member selected from the group consisting of zeaxanthin, β-cryptoxanthin, lutein, and fatty acid esters thereof, which are components corresponding to xanthophyll among normally present carotenoids.

Zeaxanthin is a xanthophyll contained in plant parts such as a corn seed and orange-colored paprika. The zeaxanthin used in the present invention may be extracted from these plant parts or chemically synthesized.

β-Cryptoxanthin is a xanthophyll contained in citruses such as orange and grapefruit. The β-cryptoxanthin used in the present invention may be extracted from these plants or chemically synthesized.

Lutein is a xanthophyll contained in plants such as leaf vegetables including spinach, kale, and Japanese mustard spinach; and petals of marigold. The lutein used in the present invention may be extracted from these plants or chemically synthesized.

In addition, zeaxanthin, β-cryptoxanthin, and lutein may be ester(s) in which fatty acid(s) is/are ester-bonded. The ester may be a monoester in which one fatty acid is ester-bonded or a diester in which two fatty acids are ester-bonded. In the case where the ester is a diester, the two fatty acids bonded may have the same structure or different structures. The structure and the like of the fatty acid bonded in the ester are the same as those of the ester used as the component (A). Hereinafter, the concept that encompasses zeaxanthin and/or a fatty acid ester thereof may be referred to as "zeaxanthins" in some cases. Likewise, with respect to β-cryptoxanthin and/or a fatty acid ester thereof, and lutein and/or a fatty acid ester thereof, similarly, they may be referred to as "β-cryptoxanthins", and "luteins", respectively, in some cases.

In the agent for improving the carotenoid balance in blood according to the present invention, as the component (B), one type selected from zeaxanthin, β-cryptoxanthin, lutein, and fatty acid esters thereof may be used or two or more types of them may be used in combination.

Among these components (B), from the viewpoint of more effectively inhibiting the decreases in the blood levels of the normally present carotenoids and increasing the blood levels of the normally non-present xanthophylls, for example, preferably, at least one type of zeaxanthins and β-cryptoxanthins is used; and more preferably a combination of zeaxanthins and β-cryptoxanthins is used.

In the case where a combination of zeaxanthins and β-cryptoxanthins is used as the component (B), the ratio of them is not particularly limited. Examples of the ratio include, per 100 parts by mass of zeaxanthins in terms of free zeaxanthin weight, 1 to 2000 parts by mass, preferably 5 to 1000 parts by mass and more preferably 10 to 500 parts by mass of β-cryptoxanthins in terms of free β-cryptoxanthin weight. In the present specification, zeaxanthins in terms of free zeaxanthin weight refers to the value of the weight of itself in the case of zeaxanthin, and in the case of fatty acid ester(s) of zeaxanthin, it refers to the value converted to the weight of zeaxanthin (free form) per se, excluding the fatty acid moiety. The same applies to β-cryptoxanthins in terms of β-cryptoxanthin weight.

In the agent for improving the carotenoid balance in blood according to the present invention, the ratio of the component (B) to the component (A) is not particularly limited, but from the viewpoint of more effectively inhibiting the decreases in the blood levels of the normally present carotenoids and increasing the blood levels of the normally non-present xanthophylls, examples of the ratio include, per 100 parts by mass of the component (A) in terms of free xanthophyll weight, 1 to 1000 parts by mass, preferably 10 to 500 parts by mass, and more preferably 5 to 200 parts by mass of the component (B) in terms of free xanthophyll weight. In the present specification, the component (A) in terms of free xanthophyll weight refers to the value of the weight of itself in the case where the component (A) is a xanthophyll per se, and in the case where the component (A) is a fatty acid ester of a xanthophyll, it refers to the value converted to the weight of xanthophyll (free form) per se, excluding the fatty acid moiety. The same applies to the component (B) in terms of xanthophyll weight.

### Component (C)

The component (C) used in the present invention is at least one member selected from the group consisting of β-carotene, α-carotene, and lycopene, which are components corresponding to carotene among normally present carotenoids.

β-Carotene is a carotene contained in plants such as carrot, parsley, red pepper, basil, mugwort, spinach, Angelica keiskei, crown daisy, shepherd's purse, Japanese radish, leek, and seaweed. The β-carotene used in the present invention may be extracted from these plants or chemically synthesized.

α-Carotene is a carotene contained in plants such as carrot, pumpkin, and seaweed. The α-carotene used in the present invention may be extracted from these plants or chemically synthesized.

Lycopene is a carotene contained in plants such as tomato, watermelon, pink grapefruit, Japanese persimmon, pink guava, and papaya. The lycopene used in the present invention may be extracted from these plants or chemically synthesized.

In the agent for improving the carotenoid balance in blood according to the present invention, as the component (C), one type selected from β-carotene, α-carotene, and lycopene may be used or two or more types of them may be used in combination.

Among these components (C), from the viewpoint of more effectively inhibiting the decreases in the blood levels of the normally present carotenoids and increasing the blood levels of the normally non-present xanthophylls, for example, preferably, at least one type of β-carotene and α-carotene is used; and more preferably β-carotene is used.

In the agent for improving the carotenoid balance in blood according to the present invention, the ratio of the component (C) to the component (A) is not particularly limited, but from the viewpoint of more effectively inhibiting the decreases in the blood levels of the normally present carotenoids and increasing the blood levels of the normally non-present xanthophylls, examples of the ratio include, per 100 parts by mass of the component (A) in terms of free xanthophyll weight, 1 to 70 parts by mass, preferably 3 to 50 parts by mass, and more preferably 5 to 30 parts by mass of the component (C).

### One Embodiment of Components (A) to (C)

In the agent for improving the carotenoid balance in blood according to the present invention, the components (A) to (C) may be individually extracted or produced. They may also be obtained by simultaneously extracting these components from plants, algae, fish and shellfish, etc. containing the components (A) to (C) as raw materials. In this case, it is desirable to use a preparation extracted, purified and processed under optimum conditions so that the components (A) to (C) have the above-described preferable ratio. Extraction of xanthophylls from plants, algae, and fish and shellfish and the like can be carried out according to a known method. In addition, as a preparation extracted, purified and processed to contain the components (A) to (C) in the above-described preferred ratio, the product name "PapriX" (manufactured by GLICO NUTRITION CO., LTD.) is commercially available. Said commercially available product can also be used as the components (A) to (C).

### Component (D)

The component (D) used in the present invention is vitamin E, which is not classified as carotenoid, but is a component which contributes to inhibiting the decreases in the blood levels of the normally present carotenoids and increasing the blood levels of the normally non-present xanthophylls.

Vitamin E is a concept including tocopherol, tocotrienol, derivatives thereof, and salts thereof.

Specific examples of tocopherol include d-α-tocopherol, d-β-tocopherol, d-γ-tocopherol, d-δ-tocopherol, l-α-tocopherol, l-β-tocopherol, l-γ-tocopherol, l-δ-tocopherol, as well as dl-α-tocopherol, dl-β-tocopherol, dl-γ-tocopherol, and dl-δ-tocopherol which are mixtures thereof and the like.

The derivatives of tocopherol are not particularly limited as long as they are edible. Examples of the derivatives of tocopherol include tocopherol acetate, tocopherol succinate, tocopherol phosphate, tocopherol aspartate, tocopherol glutamate, tocopherol palmitate, tocopherol nicotinate, tocopherol linoleate and the like.

Specific examples of tocotrienol include α-tocotrienol, β-tocotrienol, γ-tocotrienol, δ-tocotrienol and the like.

The derivatives of tocotrienol are not particularly limited as long as they are edible. Examples of the derivatives of tocotrienol include tocotrienol acetate, tocotrienol succinate, tocotrienol phosphate, tocotrienol aspartate, tocotrienol glutamate, tocotrienol palmitate, tocotrienol nicotinate and the like.

Specific examples of the vitamin E in the form of a salt include an alkali metal salt of a derivative of tocopherol (for example, a calcium salt and the like), an alkali metal salt of a derivative of tocotrienol (for example, a calcium salt and the like), and the like.

One type of the vitamin E may be used alone or two or more types of them may be used in combination.

The component (D) used in the present invention may also comprise vitamin C in addition to vitamin E. Vitamin C is a concept encompassing L-ascorbic acid and a salt thereof.

The salt of L-ascorbic acid is not particularly limited as long as it is edible but examples thereof include an alkali metal salt such as sodium salt and potassium salt; an alkaline earth metal salt such as calcium salt and magnesium salt and the like.

As the vitamin C, one type selected from L-ascorbic acid and a salt thereof may be selected and used alone, or two or more types of them may be used in combination.

A vitamin C derivative is a concept encompassing a derivative of L-ascorbic acid and a salt thereof.

As the derivative of L-ascorbic acid, there is not particular limitation as long as it is edible, and examples thereof include L-ascorbic acid glucoside (such as L-ascorbic acid-2 glucoside, and L-ascorbic acid-6 glucoside), L-ascorbic acid phosphate ester (such as L-ascorbic acid-2-phosphate ester, L-ascorbic acid-3-phosphate ester, and L-ascorbic acid-6-phosphate ester), L-ascorbic acid-2-polyphosphate ester, L-ascorbic acid-2-sulfate ester, L-ascorbic acid-2-palmitate ester, L-ascorbic acid-6-palmitate ester, L-ascorbic acid-2-stearate ester, L-ascorbic acid-6-stearate ester and the like. The salt of the derivative of L-ascorbic acid is not particularly limited as long as it is edible but examples thereof include an alkali metal salt such as sodium salt and potassium salt; an alkaline earth metal salt such as calcium salt and magnesium salt and the like.

One type of the vitamin C derivative may be used alone or two or more types of them may be used in combination in addition to vitamin E.

In the agent for improving the carotenoid balance in blood according to the present invention, as the component (D), one type of vitamin E may be used or two or more types of them may be used in combination.

Among these components (D), from the viewpoint of more effectively inhibiting the decreases in the blood levels of the normally present carotenoids and increasing the blood levels of the normally non-present xanthophylls, preferably, vitamin E alone or a combination of vitamin E and vitamin C and/or a vitamin C derivative is used. Hereinafter, the concept that encompasses vitamin C and/or vitamin C derivatives may be referred to as "vitamin Cs" in some cases.

In the case where a combination of vitamin E and vitamin Cs is used as the component (D), the ratio of them is not particularly limited. Examples of the ratio include, per 100 parts by mass of vitamin E, 100 to 20000 parts by mass, preferably 500 to 10000 parts by mass and more preferably 1000 to 5000 parts by mass of vitamin Cs in terms of free ascorbic acid weight. In the present specification, vitamin Cs in terms of free ascorbic acid weight refers to the value of the weight of itself in the case of ascorbic acid, in the case of a salt of ascorbic acid, it refers to the value converted to the weight of ascorbic acid (free form), and in the case of a derivative of ascorbic acid, it refers to the value converted to the weight of ascorbic acid per se, excluding the substituent(s) and salt(s) attached to ascorbic acid.

In the agent for improving the carotenoid balance in blood according to the present invention, the ratio of the component (D) to the component (A) is not particularly limited, but from the viewpoint of more effectively inhibiting the decreases in the blood levels of the normally present carotenoids and increasing the blood levels of the normally non-present xanthophylls, examples of the ratio include, per 100 parts by mass of the component (A) in terms of free xanthophyll weight, 1 to 10000 parts by mass of the component (D) in total of an amount of vitamin E and an amount in terms of free ascorbic acid weight. More specifically, when vitamin E is used alone as the component (D), for example, vitamin E is preferably 1 to 5000 parts by mass, more preferably 10 to 200 parts by mass and particularly preferably 20 to 100 parts by mass per 100 parts by mass of the component (A) in terms of free xanthophyll weight. In the case where a combination of vitamin E and vitamin Cs is used as the component (D), examples of the ratio thereof include, per 100 parts by mass of the component (A) in terms of free xanthophyll weight, preferably 1 to 5000 parts by mass, more preferably 10 to 200 parts by mass, and particularly preferably 20 to 100 parts by mass of vitamin E; and preferably 500 to 10000 parts by mass, more preferably 1000 to 6000 parts by mass and particularly preferably 1500 to 3000 parts by mass of vitamin Cs in terms of free ascorbic acid weight.

In the agent for improving the carotenoid balance in blood according to the present invention, the ratio of the component (D) to the total amount of the components (A) to (C) is not particularly limited, but from the viewpoint of more effectively inhibiting the decreases in the blood levels of the normally present carotenoids and increasing the blood levels of the normally non-present xanthophylls, examples of the ratio include, per 100 parts by mass of the components (A) to (C) in total in terms of free carotenoid weight, 1 to 20000 parts by mass of the component (D) in total of an amount of vitamin E and an amount in terms of free ascorbic acid weight. More specifically, when vitamin E is used alone as the component (D), for example, vitamin E is preferably 1 to 1000 parts by mass, more preferably 5 to 500 parts by mass and particularly preferably 10 to 100 parts by mass per 100 parts by mass of the components (A) to (C) in total in terms of free carotenoid weight. In the case where a combination of vitamin E and vitamin Cs is used as the component (D), examples of the ratio include, per 100 parts by mass of the components (A) to (C) in total in terms of free carotenoid weight, preferably 1 to 1000 parts by mass, more preferably 5 to 500 parts by mass, and particularly preferably 10 to 100 parts by mass of vitamin E; and preferably 100 to 20000 parts by mass, more preferably 500 to 10000 parts by mass and particularly preferably 1000 to 2000 parts by mass of vitamin Cs in terms of free ascorbic acid weight. In the present specification, the components (A) to (C) in total in terms of free carotenoid weight means the total of the value of the weight of the component (A) in terms of free xanthophyll, the value of the weight of the component (B) in terms of free xanthophyll and the value of the weight of the component (C).

### Use

The agent for improving the carotenoid balance in blood according to the present invention is capable of increasing the blood level of the component (A) which is the normally non-present xanthophylls having high antioxidant properties (scavenging activities of singlet oxygen), while inhibiting the decreases in the blood levels of the normally present carotenoids, and therefore, the agent is used for the purpose of increasing the antioxidative activity in the living body and for maintaining health and preventing diseases.

More specifically, the agent for improving the carotenoid balance in blood according to the present invention is capable of enhancing the protective action against oxidative stress of a living body by increasing the antioxidative activity of the living body, and therefore, the agent is effective for improving the physical function, and is particularly effective for recovering the physical function during or after exercise. Accordingly, the agent for improving the carotenoid balance in blood according to the present invention can be used as an agent for increasing antioxidative activities, an agent for increasing the physical function, an agent for increasing the cardiopulmonary function, an agent for recovering physical function during or after exercise and the like.

In addition, protection against oxidative stress in a living body is also effective for maintaining brain function and improving brain function, and therefore, the agent for improving the carotenoid balance in blood according to the present invention can also be used as an agent for maintaining or improving the brain function.

Further, protection against oxidative stress in a living body can increase the oxygen carrying capacity of erythrocytes, and therefore, the agent for improving the carotenoid balance in blood according to the present invention is used according to the invention as an agent for preventing or treating dementia such as Alzheimer-type dementia, arteriosclerosis, hypertension, diabetes, hyperlipidemia, cataract, and cerebral stroke.

### Usage form of agent for improving carotenoid balance in blood

The application form of the agent for improving the carotenoid balance in blood according to the present invention is not particularly limited as long as it is an embodiment where the agent is absorbed into a living body, and examples thereof include oral, enteral, intravenous, transarterial, subcutaneous, intramuscular and the like. Among these application forms, from the viewpoint of convenience, efficiency, etc., a preferable example is oral application.

The agent for improving the carotenoid balance in blood according to the present invention can be provided in the form of a commodity such as a food or beverage, a pharmaceutical preparation and the like.

The dosage form of the agent for improving the carotenoid balance in blood according to the present invention is not particularly limited and may be any of solid, semisolid, liquid and the like, and may be appropriately set depending on the type and application of the form of the commodity of the agent.

In addition, the agent for improving the carotenoid balance in blood according to the present invention may contain, in addition to the components described above, a food material, a food additive, a nutritional component, a pharmaceutically acceptable base material, a pharmaceutically acceptable additive, a pharmacological component and the like, depending on the type, the dosage form and the like of the form of the commodity of the agent, as long as the effect of the present invention is not impaired.

Further, the agent for improving the carotenoid balance in blood according to the present invention may contain carotenoid(s) other than the components (A) to (C) described above. When carotenoid(s) other than the components (A) to (C) is/are contained in the agent for improving the carotenoid balance in blood according to the present invention, it is desirable that, per 100 parts by mass of the total amount in terms of free carotenoid weight contained in the agent for improving the carotenoid balance in blood according to the present invention, the components (A) to (C) in total is 0.01 parts by mass or more, preferably 0.1 parts by mass or more, more preferably 30 parts by mass or more, even more preferably 50 parts by mass of more, and particularly preferably 70 parts by mass or more, in terms of free carotenoid weight.

In the case where the agent for improving the carotenoid balance in blood according to the present invention is provided in the form of a food or beverage, the components (A) to (D) described above may be prepared, as they are or in combination with other food materials and additive components, into a desired form. Examples of such foods or beverages include special health foods, nutritional supplementary foods, functional foods, foods for patients and the like, in addition to general foods or beverages. The form of these foods or beverages is not particularly limited, and specific examples include staple foods such as baked goods and noodles; side dishes such as cheese, ham, Vienna sausages and processed fish and shellfish products; confectioneries such as gum, chocolates, soft candies, hard candies, gummi candies, biscuits, cookies, crackers, Okaki (fried rice crackers), rice crackers and puffed snacks; ice confectioneries such as ice creams, soft ice creams, sherbets and frozen desserts; supplements and the like such as tablets, granules, powders, hard capsules, soft capsules, and gummi candies; drinks such as soft drinks, milk-based drinks, fermented milk drinks, carbonated drinks, fruit juice drinks, vegetable drinks, vegetable and fruit drinks, powder drinks, jelly drinks, coffee drinks, black tea drinks, green tea drinks, sport drinks, fortified drinks, energy drinks, non-alcoholic drinks, and alcoholic drinks, and the like.

Among these foods or beverages, preferred are supplements (tablets, granules, powders, hard capsules, soft capsules, gummi candies, etc.) and drinks (especially fortified drinks and energy drinks) from the viewpoints of convenience of ingestion and efficiency and the like.

In addition, in the case where the agent for improving the carotenoid balance in blood according to the present invention is provided as a pharmaceutical preparation, the components (A) to (D) described above alone or in combination with other pharmacological components, pharmaceutically acceptable base materials, additives and the like, may be prepared into a desired dosage form. The form of such a pharmaceutical preparation is not particularly limited, but specifically, examples include oral pharmaceutical preparations such as tablets, granules, powders, capsules, soft capsules and syrups; pharmaceutical preparations for systemic administration such as injections and infusions and the like. Among them, oral pharmaceutical preparations are preferred.

### Application amount and content of (A) to (D)

Regarding the application amount of the agent for improving the carotenoid balance in blood according to the present invention, it may be an effective amount for increasing the blood level of the component (A), which is normally non-present xanthophyll(s), while inhibiting the decreases in the blood levels of the normally present carotenoids, and it may be appropriately set depending on the type and form of the product to be used, the application, the expected effect, the application form, and the like.

For example, the application amount may be set such that an intake or a dose per day for an adult is 0.05 to 40 mg, preferably 0.3 to 30 mg, more preferably 0.5 to 17 mg, and particularly preferably 0.8 to 11 mg of the component (A) in terms of free xanthophyll weight.

In addition, for example, the application amount may be set such that an intake or a dose per day for an adult is 0.01 to 10 mg, preferably 0.07 to 7 mg, more preferably 0.1 to 4 mg, and preferably 0.3 to 3 mg of the component (B) in terms of free xanthophyll weight.

Further, for example, the application amount may be set such that an intake or a dose per day for an adult is 0.01 to 10 mg, preferably 0.07 to 7 mg, more preferably 0.1 to 4 mg, and preferably 0.3 to 3 mg of the component (C).

Furthermore, for example, the application amount may be set such that an intake or a dose per day for an adult is 0.01 to 2000 mg of the component (D) in total of an amount of vitamin E and an amount in terms of free ascorbic acid weight. More specifically, when vitamin E is used alone as the component (D), for example, an intake or a dose per day for an adult of vitamin E is preferably 0.01 to 500 mg, more preferably 0.1 to 300 mg and particularly preferably 1 to 50 mg. In the case where a combination of vitamin E and vitamin Cs is used as the component (D), for example, an intake or a dose per day for an adult of vitamin E is preferably 0.01 to 500 mg, more preferably 0.1 to 300 mg and particularly preferably 1 to 50 mg; and an intake or a dose of vitamin Cs is preferably 10 to 2000 mg, more preferably 50 to 1500 mg, and particularly preferably 100 to 1000 mg in terms of free ascorbic acid weight.

In the agent for improving the carotenoid balance in blood according to the present invention, the contents of the components (A) to (D) may be appropriately set within a range that can satisfy the ratio of each component and the application amount described above according to the form of the commodity. For example, in the agent for improving the carotenoid balance in blood according to the present invention, the content of the component (A) is 0.0001 to 60 mass%, and preferably 0.001 to 10 mass% in terms of free xanthophyll weight; the content of the component (B) is 0.0001 to 10 mass%, and preferably 0.001 to 1 mass% in terms of free xanthophyll weight; the content of the component (C) is 0.0001 to 10 mass%, and preferably 0.001 to 1 mass%; and the content of the component (D) is 0.0001 to 20 mass%, and preferably 0.001 to 10 mass% in total of an amount of vitamin E and an amount in terms of free ascorbic acid weight.

More specifically, when the agent for improving the carotenoid balance in blood according to the present invention is a supplement, for example, the content of the component (A) is 0.1 to 60 mass%, and preferably 1 to 10 mass% in terms of free xanthophyll weight; the content of the component (B) is 0.02 to 10 mass%, and preferably 0.1 to 1 mass% in terms of free xanthophyll weight; the content of the component (C) is 0.02 to 10 mass%, and preferably 0.1 to 1 mass%; and the content of the component (D) is 0.1 to 20 mass%, and preferably 1 to 10 mass% in total of an amount of vitamin E and an amount in terms of free ascorbic acid weight.

In addition, when the agent for improving the carotenoid balance in blood according to the present invention is a drink, for example, the content of the component (A) is 0.0001 to 0.1 mass%, and preferably 0.001 to 0.01 mass% in terms of free xanthophyll weight; the content of the component (B) is 0.0001 to 0.1 mass%, and preferably 0.001 to 0.01 mass% in terms of free xanthophyll weight; the content of the component (C) is 0.0001 to 0.1 mass%, and preferably 0.001 to 0.01 mass%; and the content of the component (D) is 0.0001 to 10 mass%, and preferably 0.001 to 10 mass% in total of an amount of vitamin E and an amount in terms of free ascorbic acid weight.

### 2. Food or beverage

The food or beverage according to the present invention comprises: (A) at least one member selected from the group consisting of capsorubin, capsanthin, capsanthone, cucurbitaxanthin A, astaxanthin, mytiloxanthin, and fatty acid esters thereof; (B) at least one member selected from the group consisting of lutein, zeaxanthin, β-cryptoxanthin, and fatty acid esters thereof; (C) at least one member selected from the group consisting of β-carotene, α-carotene, and lycopene; and (D) vitamin E.

The types of the components (A) to (D) used in the food or beverage according to the present invention, their ratios, intake amounts, contents, forms and the like are as described in paragraphs [0014] to [0081], see "1. Agent for improving carotenoid balance in blood".

The purpose of ingesting the food or beverage according to the present invention is not particularly limited, but it is preferably used for improving the carotenoid balance in blood. Specific examples of applications for improving the carotenoid balance in blood are also as described above under "1. Agent for improving carotenoid balance in blood".

### EXAMPLES

Hereinafter, the present invention will be specifically described with reference to Examples, but the present invention is not limited to these Examples.

### Reference Example 1 (Prior Art)

Hereinafter, experimental data shown in Non-Patent Document 2 will be described. In Non-Patent Document 2, a capsule having the composition shown in Table 1 has been made to be ingested in 10 healthy subjects at a frequency of 1 capsule per day for 28 days, and the carotenoid concentration in erythrocytes before ingestion and after ingesting for 28 days has been measured by using HPLC.

**[Table 1]**

| Ingredient | Mass per 1 capsule (content) (mg) | Intake amount per day (mg) |
|---|---|---|
| (A) Haematococcus pluvialis extract containing astaxanthin | 60 (Containing 3 mg of astaxanthin) | 60 (Containing 3 mg of astaxanthin) |
| Olive oil | 160 | 160 |
| Vitamin E | 30 | 30 |
| Total | 250 | 250 |

The results shown in Non-Patent Document 2 are shown in Table 2. When astaxanthin has been made to be ingested alone, although the concentration of astaxanthin in erythrocytes has been increased, the concentrations of β-cryptoxanthin, lutein and zeaxanthin which are normally present carotenoids in erythrocytes have remarkably decreased, and a significant decrease in the total carotenoid concentration in erythrocytes has also been observed.

**[Table 2]**

| | Concentration in erythrocytes (pmol/mL) | |
|---|---|---|
| | Before ingestion | After ingesting for 28 days |
| Astaxanth in | 0.9 | 2.5 |
| β-Carotene | 4.5 | 3.9 |
| β-Cryptoxa nthin | 32.5 | 14.8 |
| Lutein | 59.7 | 23.2 |
| Zeaxanthi n | 10.6 | 3.7 |
| Total carotenoi ds | 107.3 | 48.1 |

### Excerpt from results in Non-Patent Document 2

### Reference Example 2 (Prior Art)

Hereinafter, experimental data shown in Non-Patent Document 3 will be described. In Non-Patent Document 3, a capsule having the composition shown in Table 3 has been made to be ingested in 6 healthy subjects at a frequency of 1 capsule per day for 28 days, and the plasma carotenoid concentration before ingestion and after ingesting for 28 days has been measured by using HPLC.

**[Table 3]**

| Ingredient | Mass per 1 capsule (content) (mg) | Intake amount per day (mg) |
|---|---|---|
| (B) Lutein^{#} | 9.67 | 9.67 |
| (B) Zeaxanthin^{#} | 0.73 | 0.73 |
| α-Tocopherol | 0.12 | 0.12 |
| Total | 10.52 | 10.52 |

| | | |
|---|---|---|
| # In Non-Patent Document 3, marigold extract has been used as source of lutein and zeaxanthin. | | |

The results shown in Non-Patent Document 3 are shown in Table 4. When a combination of lutein and zeaxanthin has been made to be ingested, although the plasma concentration of lutein has been increased, the plasma concentrations of β-carotene, α-carotene, and β-cryptoxanthin which are normally present carotenoids have remarkably decreased, and further, there has been little change in the total plasma carotenoid concentration.

**[Table 4]**

| | Average concentration in erythrocytes (pmol/mL) | |
|---|---|---|
| | Before ingestion | After ingesting for 28 days |
| Lutein | 341 | 768 |
| β-Carotene | 475 | 337 |
| α-Carotene | 184 | 135 |
| Lycopene | 303 | 301 |
| β-Cryptoxanthin | 411 | 224 |
| Zeaxanthin | 115 | 120 |
| Total carotenoids | 1829 | 1885 |

### Excerpt from results in Non-Patent Document 3

### Reference Example 3 (Prior Art)

Hereinafter, experimental data shown in Non-Patent Document 4 will be described. In Non-Patent Document 4, a drink having the composition shown in Table 5 has been made to be ingested in 5 healthy subjects in the morning and in the evening in the total amount of 200 ml per day for 28 days, and the plasma carotenoid concentration before ingestion and after ingesting for 28 days has been measured by using HPLC.

**[Table 5]**

| Ingredient | | Mass per 1 L (mg) | Intake amount per day (mg) |
|---|---|---|---|
| Preparation of paprika extract^{#} | | 7,000 | 7,000 |
| Xanthophylls contained in preparation of paprika extract | (A) Capsanthin | 32.7 | 6.5 |
| | (A) Capsorubin | 1.8 | 0.4 |
| | (A) Cucurbitaxanthin A | 5.7 | 1.1 |
| | (B) β-Cryptoxanthin | 5.4 | 1.1 |
| | (B) Zeaxanthin | 4.6 | 0.92 |
| | (C) β-Carotene | 9.0 | 1.8 |
| | Cryptocapsin | 0.7 | 0.14 |
| | Capsanthin epoxide | 2.7 | 0.54 |
| | Other xanthophylls (unidentified) | 7.4 | 1.5 |
| Granulated sugar | | 70,000 | 14,000 |
| Citric acid | | 2,000 | 400 |
| Sodium citrate | | 1,000 | 200 |
| Purified water | | Remainder | - |

| | | | |
|---|---|---|---|
| # "PapriX" manufactured by GLICO NUTRITION CO., LTD. has been used as preparation of paprika extract. | | | |

The results shown in Non-Patent Document 4 are shown in Table 6. When normally present carotenoids (xanthophyll and carotene) have been made to be ingested along with normally non-present xanthophylls, although increases in the plasma concentrations of the normally non-present xanthophylls have been observed, remarkable decreases in the plasma concentrations of the total carotenes, and lutein, which are normally present carotenoids, have been observed.

**[Table 6]**

| | Average plasma concentration (pmol/mL) | |
|---|---|---|
| | Before ingestion | After ingesting for 28 days |
| Total amount of capsanthin and capsorubin | Undetected^{#} | 124.7 |
| Cucurbitaxanthin A | Undetected^{#} | 111.8 |
| Cryptocapsin | Undetected^{#} | 8.2 |
| Total carotenes | 1272.9 | 1101.7 |
| β-Cryptoxanthin | 259.0 | 547.3 |
| Lutein | 322.0 | 232.1 |
| Zeaxanthin | 53.9 | 100.9 |
| Total carotenoids | 1907.8 | 2226.7 |

| | | |
|---|---|---|
| # Undetected indicates determination limit or less. | | |

### Excerpt from results in Non-Patent Document 4

### Example 1

A drink having the composition shown in Table 7 was produced and changes in plasma carotenoids by ingestion of the drink were measured. Specifically, one healthy subject was made to ingest 100 ml of the drink per ingestion once a day for 28 days. Blood was collected before ingestion and after ingesting for 28 days to obtain plasma, and the plasma concentration of each carotenoid was quantified by HPLC.

**[Table 7]**

| | Ingredient | Mass per 1 L (mg) | Intake amount per day (mg) |
|---|---|---|---|
| Preparation of paprika extract^{#} | | 14,000 | 1,400 |
| Xanthophylls contained in preparation of paprika extract | (A) Capsanthin | 65.0 | 6.5 |
| | (A) Capsorubin | 4.0 | 0.4 |
| | (A) Cucurbitaxanthin A | 11.0 | 1.1 |
| | (B) β-Cryptoxanthin | 11.0 | 1.1 |
| | (B) Zeaxanthin | 9.0 | 0.9 |
| | (C) β-Carotene | 18.0 | 1.8 |
| | Cryptocapsin | 1 | 0.1 |
| | Capsanthin epoxide | 5 | 0.5 |
| | Other xanthophylls (unidentified) | 15 | 1.5 |
| Mixed tocopherols ^{#2} | | 100 | 10 |
| Vitamin E contained in mixed tocopherols | (D) Vitamin E | 50 | 5 |
| (D) L-ascorbic acid | | 2,000 | 200 |
| Citric acid | | 800 | 80 |
| Sodium citrate | | 600 | 60 |
| Erythritol | | 10,000 | 1,000 |
| Acesulfame potassium | | 300 | 30 |
| Purified water | | Remainder | - |

| | | | |
|---|---|---|---|
| #1 "PapriX" manufactured by GLICO NUTRITION CO., LTD. was used as preparation of paprika extract. #2 As mixed tocopherols, those of "Riken E Oil 600" manufactured by RIKEN VITAMIN CO., LTD. were used. | | | |

The obtained results are shown in Table 8. As a result, when vitamin E and vitamin C were made to be ingested together with normally non-present xanthophylls and normally present carotenoids (xanthophylls and carotene), plasma concentrations of normally non-present xanthophylls were remarkably increased and the total carotenoid concentration in blood was significantly increased without decreasing the plasma concentrations of normally present carotenoids (xanthophylls and carotenes). In addition, among the normally present carotenoids, the plasma concentration of β-cryptoxanthin was significantly increased.

**[Table 8]**

| | Average plasma concentration (pmol/mL) | |
|---|---|---|
| | Before ingestion | After ingesting for 28 days |
| Total amount of capsanthin and capsorubin | Undetected^{#} | 36.8 |
| Cucurbitaxanthin A | 45.5 | 74.2 |
| Total carotenes | 785.3 | 864.3 |
| β-Cryptoxanthin | 193.7 | 680.7 |
| Cryptocapsin | Undetected^{#} | 16.0 |
| Lutein | 178.1 | 267.0 |
| Zeaxanthin | 12.5 | 56.2 |
| Total carotenoids | 1224.6 | 1995.3 |

| | | |
|---|---|---|
| # Undetected indicates determination limit or less. | | |

### Example 2

A capsule (hard capsule) having the composition shown in Table 9 was produced and changes in plasma carotenoids by ingestion of the capsule were measured. Specifically, one healthy subject was made to ingest 1 tablet per ingestion once a day for 28 days. Blood was collected before ingestion and after ingesting for 28 days to obtain plasma, and the plasma concentration of each carotenoid was quantified by HPLC.

**[Table 9]**

| | Ingredient | Mass per 1 capsule (content) (mg) | Intake amount per day (mg) |
|---|---|---|---|
| Preparation of paprika extract^{#} | | 330 | 330 |
| Xanthophylls contained in preparation of paprika extract | (A) Capsanthin | 4.7 | 4.7 |
| | (A) Capsorubin | 0.3 | 0.3 |
| | (A) Cucurbitaxanthin A | 0.8 | 0.8 |
| | (B) β-Cryptoxanthin | 0.8 | 0.8 |
| | (B) Zeaxanthin | 0.7 | 0.7 |
| | (C) β-Carotene | 1.3 | 1.3 |
| | Cryptocapsin | 0.1 | 0.1 |
| | Capsanthin epoxide | 0.4 | 0.4 |
| | Other xanthophylls (unidentified) | 1.1 | 1.1 |
| Mixed tocopherols ^{#2} | | 10 | 10 |
| Vitamin E contained in mixed tocopherols | (D) Vitamin E | 5 | 5 |
| Total | | 340 | - |

| | | | |
|---|---|---|---|
| #1 "PapriX" manufactured by GLICO NUTRITION CO., LTD. was used as preparation of paprika extract. #2 As mixed tocopherols, those of "Riken E Oil 600" manufactured by RIKEN VITAMIN CO., LTD. were used. | | | |

The obtained results are shown in Table 10. As a result also, when vitamin E and vitamin C were made to be ingested together with normally non-present xanthophylls and normally present carotenoids (xanthophylls and carotene), plasma concentrations of normally non-present xanthophylls were remarkably increased and the total carotenoid plasma concentration was significantly increased without decreasing the plasma concentrations of normally present carotenoids. In addition, in the same manner as Example 1, among the normally present carotenoids, the plasma concentration of β-cryptoxanthin was significantly increased.

**[Table 10]**

| | Average plasma concentration (pmol/mL) | |
|---|---|---|
| | Before ingestion | After ingesting for 28 days |
| Total amount of capsanthin and capsorubin | Undetected^{#} | 77.8 |
| Cucurbitaxanthin A | Undetected^{#} | 117.2 |
| Total carotenes | 1703.7 | 1725.1 |
| β-Cryptoxanthin | 114.9 | 496.2 |
| Cryptocapsin | Undetected^{#} | 31.7 |
| Lutein | 223.6 | 212.1 |
| Zeaxanthin | 45.3 | 84.0 |
| Total carotenoids | 2087.5 | 2744.1 |

| | | |
|---|---|---|
| # Undetected indicates determination limit or less. | | |

## Claims

1. Agent comprising:
(A) at least one member selected from the group consisting of capsanthin, capsorubin, capsanthone, cucurbitaxanthin A, astaxanthin, mytiloxanthin, and fatty acid esters thereof;
(B) at least one member selected from the group consisting of zeaxanthin, β-cryptoxanthin, lutein, and fatty acid esters thereof;
(C) at least one member selected from the group consisting of β-carotene, α-carotene, and lycopene; and
(D) vitamin E,
for use in a method of treating dementia, arteriosclerosis, hypertension, diabetes, hyperlipidemia, cataract, and cerebral stroke.

2. Agent for use in a method according to claim 1, wherein the component (A) is at least one member selected from the group consisting of capsanthin, capsorubin, capsanthone, cucurbitaxanthin A, and fatty acid esters thereof.

3. Agent for use in a method according to claim 1 or 2, wherein the component (A) is a combination of capsanthin and/or a fatty acid ester thereof, capsorubin and/or a fatty acid ester thereof, and cucurbitaxanthin A and/or a fatty acid ester thereof.

4. Agent for use in a method according to any one of claims 1 to 3, wherein the component (B) is at least one member selected from the group consisting of zeaxanthin, β-cryptoxanthin, and fatty acid esters thereof.

5. Agent for use in a method according to any one of claims 1 to 4, wherein the component (B) is a combination of zeaxanthin and/or a fatty acid ester thereof, and β-cryptoxanthin and/or a fatty acid ester thereof.

6. Agent for use in a method according to any one of claims 1 to 5, wherein the component (C) is β-carotene.

7. Agent for use in a method according to any one of claims 1 to 6, wherein the agent comprises vitamin C and/or vitamin C derivatives.

8. Agent for use in a method according to any one of claims 1 to 7, the agent comprising:
0.0001 to 60 mass% of the component (A) in total in terms of free xanthophyll weight;
0.0001 to 10 mass% of the component (B) in total in terms of free xanthophyll weight;
0.0001 to 10 mass% of the component (C) in total; and
0.0001 to 20 mass% of the component (D) in total of an amount of vitamin E and an amount in terms of free ascorbic acid weight.

9. Agent for use in a method according to any one of claims 1 to 8, which is a food or beverage.

## Patentansprüche

1. Mittel umfassend:
(A) mindestens ein Mitglied ausgewählt aus der Gruppe bestehend aus Capsanthin, Capsorubin, Capsanthon, Cucurbitaxanthin A, Astaxanthin, Mytiloxanthin und Fettsäureester davon;
(B) mindestens ein Mitglied ausgewählt aus der Gruppe bestehend aus Zeaxanthin, β-Cryptoxanthin, Lutein und Fettsäureester davon;
(C) mindestens ein Mitglied ausgewählt aus der Gruppe bestehend aus β -Carotin,α-Carotin und Lycopin; und
(D) Vitamin E,
zur Verwendung in einem Verfahren zur Behandlung von Demenz, Arteriosklerose, Bluthochdruck, Diabetes, Hyperlipidämie, Grauem Star und zerebralem Schlaganfall.

2. Mittel zur Verwendung in einem Verfahren nach Anspruch 1, wobei die Komponente (A) mindestens ein Mitglied, ausgewählt aus der Gruppe bestehend aus Capsanthin, Capsorubin, Capsanthon, Cucurbitaxanthin A und Fettsäureestern davon, ist.

3. Mittel zur Verwendung in einem Verfahren nach Anspruch 1 oder 2, wobei die Komponente (A) eine Kombination aus Capsanthin und/oder einem Fettsäureester davon, Capsorubin und/oder einem Fettsäureester davon und Cucurbitaxanthin A und/oder einem Fettsäureester davon ist.

4. Mittel zur Verwendung in einem Verfahren nach einem der Ansprüche 1 bis 3, wobei die Komponente (B) mindestens ein Mitglied, ausgewählt aus der Gruppe bestehend aus Zeaxanthin, β-Cryptoxanthin und Fettsäureestern davon, ist.

5. Mittel zur Verwendung in einem Verfahren nach einem der Ansprüche 1 bis 4, wobei die Komponente (B) eine Kombination aus Zeaxanthin und/oder einem Fettsäureester davon, und β -Cryptoxanthin und/oder einem Fettsäureester davon, ist.

6. Mittel zur Verwendung in einem Verfahren nach einem der Ansprüche 1 bis 5, wobei die Komponente (C) β-Carotin ist.

7. Mittel zur Verwendung in einem Verfahren nach einem der Ansprüche 1 bis 6, wobei das Mittel Vitamin C und/oder Vitamin-C-Derivate umfasst.

8. Mittel zur Verwendung in einem Verfahren nach einem der Ansprüche 1 bis 7, wobei das Mittel umfasst:
0,0001 bis 60 Massen-% des Bestandteils (A) insgesamt, bezogen auf das Gewicht des freien Xanthophylls;
0,0001 bis 10 Massen-% des Bestandteils (B) insgesamt, bezogen auf das Gewicht des freien Xanthophylls;
0,0001 bis 10 Masse-% der Komponente (C) insgesamt; und
0,0001 bis 20 Massen-% des Bestandteils (D) insgesamt von einer Menge an Vitamin E und einer Menge bezogen auf das Gewicht der freien Ascorbinsäure.

9. Mittel zur Verwendung in einem Verfahren nach einem der Ansprüche 1 bis 8, das ein Lebensmittel oder ein Getränk ist.

## Revendications

1. Agent comprenant :
A) au moins un élément choisi dans l'ensemble constitué par les capsanthine, capsorubine, capsanthone, cucurbitaxanthine A, astaxanthine et mytiloxanthine, et leurs esters d'acide gras,
B) au moins un élément choisi dans l'ensemble constitué par les zéaxanthine, β-cryptoxanthine et lutéine, et leurs esters d'acide gras,
C) au moins un élément choisi dans l'ensemble constitué par les β-carotène, α-carotène et lycopène,
D) et de la vitamine E,
pour utilisation dans un procédé de traitement d'une démence, de l'artériosclérose, de l'hypertension, d'un diabète, d'une hyperlipidémie, de la cataracte ou d'une attaque cérébrale.

2. Agent pour utilisation dans un procédé, conforme à la revendication 1, dans lequel le composant (A) est au moins un élément choisi dans l'ensemble constitué par les capsanthine, capsorubine, capsanthone et cucurbitaxanthine A, et leurs esters d'acide gras.

3. Agent pour utilisation dans un procédé, conforme à la revendication 1 ou 2, dans lequel le composant (A) est une combinaison de capsanthine et/ou d'un ester d'acide gras de celle-ci, de capsorubine et/ou d'un ester d'acide gras de celle-ci, et de cucurbitaxanthine A et/ou d'un ester d'acide gras de celle-ci.

4. Agent pour utilisation dans un procédé, conforme à l'une des revendications 1 à 3, dans lequel le composant (B) est au moins un élément choisi dans l'ensemble constitué par les zéaxanthine et β-cryptoxanthine, et leurs esters d'acide gras.

5. Agent pour utilisation dans un procédé, conforme à l'une des revendications 1 à 4, dans lequel le composant (B) est une combinaison de zéaxanthine et/ou d'un ester d'acide gras de celle-ci, et de β-cryptoxanthine et/ou d'un ester d'acide gras de celle-ci.

6. Agent pour utilisation dans un procédé, conforme à l'une des revendications 1 à 5, dans lequel le composant (C) est du β-carotène.

7. Agent pour utilisation dans un procédé, conforme à l'une des revendications 1 à 6, lequel agent comprend de la vitamine C et/ou des dérivés de vitamine C.

8. Agent pour utilisation dans un procédé, conforme à l'une des revendications 1 à 7, lequel agent comprend
- de 0,0001 à 60 % en masse du composant (A) au total, en termes de poids de xanthophylles libres,
- de 0,0001 à 10 % en masse du composant (B) au total, en termes de poids de xanthophylles libres,
- de 0,0001 à 10 % en masse du composant (C) au total,
- et de 0,0001 à 20 % en masse du composant (D), total de la quantité de vitamine E et de la quantité, en poids, d'acide ascorbique libre.

9. Agent pour utilisation dans un procédé, conforme à l'une des revendications 1 à 8, qui est un aliment ou une boisson.
